(19) Europäisches Patentamt
European Patent Office
Office européen des brevets

(11) **EP 1 785 898 A1**

(12) **EUROPEAN PATENT APPLICATION**

(43) Date of publication:
**16.05.2007 Bulletin 2007/20**

(51) Int Cl.:
***G06F 19/00*** (2006.01)   *C10M 125/00* (2006.01)
***G01L 3/00*** (2006.01)

(21) Application number: **06076977.5**

(22) Date of filing: **03.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **04.11.2005 EP 05077541**

(71) Applicant: **Avantium International B.V.**
**1014 BV Amsterdam (NL)**

(72) Inventor: **McKay, Benjamin**
**1071 AT  Amsterdam (NL)**

(74) Representative: **de Lang, Robbert-Jan**
**Exter Polak & Charlouis B.V.,**
**P.O. Box 3241**
**2280 GE  Rijswijk (NL)**

(54) **Predictive technologies for lubricant development**

(57)  In a method for predicting and/or correlating additive chemical structure to engine performance, at least one molecular descriptor for one or more additives are used to characterize the chemical structure. Based on the selected molecular descriptors, a value or functional relation of model parameters of a mechanistic engine model for the engine performance e.g. using reaction kinetics parameters is determined. For example, a functional relation between the molecular descriptors and the kinetic parameters by testing compositions comprising one or more additives is determined. Based on the mechanistic engine model a QSPR library describing the relation between the molecular descriptors of the additives and the kinetic parameters an engine performance of other additives may be estimated.

Fig. **2A**

EP 1 785 898 A1

**Description**

**FIELD OF THE INVENTION**

The present invention relates to methods for the efficient and high throughput development of lubricants and/or fuels for engines. The invention in particular relates to methods for predicting and/or correlating the structure of possible additives to lubricant and/or fuel performance in engines and to provide for methods that allow the prediction of lubricant and/or fuel performance in bench tests and/or engine tests and/or engines based on molecular descriptors derived from additives and/or to provide for methods that allow selection of potential additives.

**BACKGROUND OF THE INVENTION**

In the lubricant industry, the drive toward zero emission, high energy-efficient engines demands new materials and innovative technologies to meet these goals. Another important driver for innovation is the increased environmental legislation for existing engines. New engine development is an expensive and time-consuming undertaking. Oftentimes, the long-term durability of the engine is controlled by the materials used, the mechanical design, and the effectiveness of the lubrication.

Historically, bench simulation tests are used to evaluate materials in combination with existing lubricants. Single-cylinder "bench tests" incorporating new materials or designs are conducted on an engine dynamometer to screen materials and designs. If successful, the materials and design are then tested in a prototype engine. If the material or design is significantly different from past experience, then a full-scale field test will be conducted to evaluate the long-term durability and material compatibility. Long-term lubricant-materials compatibility and durability issues often emerge at the last stage. This is expensive. Conversely, given an engine design, what kind of lubricant is needed to achieve satisfactory performance is difficult to ascertain at the beginning.

From the lubricant developers' perspective, since the new engine is evolving, many parameters have not been set and therefore it is difficult to define the lubricant needs for new engines. This leaves the lubricant developers with very little data to proceed with until the engine is sufficiently developed so that it can be made available for lubricant testing. At this time, however, many of the parameters including the materials have been fixed, and optimum lubrication may or may not be feasible.

Another issue is the warranty costs associated with the current production engines. The use of proper lubricants for a given engine design and operating conditions is vital in controlling the warranty costs. What lubricant is best for each engine operation and at what performance level the lubricant needs to be are issues the current engine manufacturers face. Current engine tests designed to qualify lubricants are becoming increasingly costly and complicated. Proper laboratory bench tests coupled with an analytical model can serve as a valuable aid to reduce the guesswork associated with the lubricant qualification testing.

An analytical model that is capable of predicting lubricant performance based on properties of lubricants and materials will significantly alleviate some of these issues.

To significantly reduce additive development timelines and reduce the number of laboratory bench tests and engine tests would be advantageous, as they require the synthesis of large amount of additives to be tested and require prolonged and expensive testing. The reduction of the amount of bench tests, although in general cheaper and faster than engine tests, is also considered advantageous as they usually do not correlate very well (i.e. have a low predictive value) with the actual engine tests. Accordingly, there is a need to make better use of the experimental data and a need for faster and more reliable testing strategies using high throughput experimentation in combination with advanced design of experiments.

In a known method for predicting chemical structure behaviour in a chemical reaction, a statistical, i.e. a purely mathematical model is employed. The statistical model comprises a predetermined number of parameters. Using test results of known chemical structures, a value for each of the parameters is determined using parameter estimation methods, e.g. Quantitative Structure-Activity Relationship (QSAR) or Quantitative Structure-Performance Relationship (QSPR). The statistical model, however, does not include any information about the chemical and physical processes. For example, if applied to using a lubricant additive, or similar chemical structure, in an engine, no insight into the chemical processes in the engine is obtained. Therefore, no insight into the working of the additive structure in the engine is obtained. Consequently, performing the known method does not improve the insight in the engine operation and/or the working of the additive structures in the engine.

**SUMMARY OF THE INVENTION**

The present inventors have now found that experimentation, whether laboratory bench testing or engine testing, of lubricants and lubricant additives can significantly be reduced by using an at least partly mechanistic engine model comprising at least one engine model parameter. A mechanistic engine model as such, or at least a part thereof, is based on technical considerations of the operation of an engine. The engine model parameter represents an engine operating parameter.

An engine operating parameter, as used herein, refers to at least one of a group comprising a property of an operating engine; a property of a chemical, physical or physiochemical process occurring in the operating engine; a condition of the operating engine; and the like; which property or condition may be represented by a value or number, or the like. A model parameter, as used herein, refers to any parameter comprised in the mechanistic engine model. The model parameter relates to at least one operating parameter and thus represents one or more engine operating parameters.

Additive structure, or additive, as used herein, refers to the chemical structure of a compound added to a lubricant or fuel formulation for the purpose of modifying the physiochemical properties of the formulation and thereby altering its performance in the engine or its stability in storage. If the additive is used to improve the performance of the stability on storage, or the like, the present invention is advantageously used to determine whether the engine performance does not deteriorate due to the additive.

The at least partly mechanistic engine model may comprise a kinetic model part representing at least one chemical reaction or physical interaction occurring in the engine when operating. Thus, the mechanistic engine model may be at least partly a representation of the chemical and/or physical processes in the engine. Moreover, any engine model parameter comprised in the kinetic model part is related to one or more of these processes. Thus, a difference in engine model parameter value between a number of additives provides information about the working of the additives in the engine. If more than one process is represented in the kinetic model part, the differences between the additives may give an indication about a possibly advantageous combination of additives, or the like.

The mechanistic engine model is configured to provide a value of a performance parameter of an engine. The performance parameter may relate to any performance aspect of the engine. For example, the performance parameter may relate to engine emission, engine power, engine durability, and the like.

In order to determine the performance parameter value, the engine model parameters need to be determined. The at least one engine model parameter may depend on at least one property of the additive, or additives, used. A property of a chemical structure may be represented by a molecular parameter, also known in the art as a molecular descriptor. A large number of molecular descriptors are known. Therefore, in the method according to the present invention, a number of molecular descriptors are selected from the large number of known molecular descriptors. Preferably, the molecular descriptors are selected based on their relevance in relation to the engine performance. The relevance may be known from the prior art, from a handbook, but may as well be determined by performing suitable experiments, use of modeling, e.g. statistical modeling like QSAR / QSPR, and/or another suitable method.

It is noted that the at least one engine model parameter may as well depend on specific engine characteristics, chemical process characteristics and/or physical process characteristics. For example, an engine model parameter may depend on a material of which the engine, or a part thereof, is constructed, an operation temperature, a concentration of one or more of the chemical structures.

Further, the operation temperature, for example, may depend on the chemical structures used. Also, other functional relations between operating parameters may exist. Therefore, it is noted that an engine model parameter may be a function of one or more operating parameters. Likewise, an engine operating parameter may be a function of one or more engine model parameters. In general, a function of one or more engine model parameters may be equal to a function of one or more engine operating parameters.

Further, in order to determine the engine model parameter value(s), a training set of additives is selected. The training set may be any random selection of chemical structures, but preferably the training set is selected such that a wide variety of potentially suitable additives is available in the training set. Using such a training set is advantageously used for obtaining an estimated engine model parameter value suitable for use with a large number of potential additives.

For each of the selected additives in the training set, the corresponding molecular descriptor values and/or corresponding parameters, as mentioned above, are collected from databases or are experimentally determined. Further, the corresponding engine performance parameter values are collected from databases or are experimentally determined, preferably for a range of operating conditions.

In order to provide a model parameter value of each engine model parameter for each chemical structure of the training set, experiments such as bench tests may be performed. As well, a suitable statistical modeling and/or estimation method, such as QSPR, may be used. In the latter case, the molecular descriptors of the additives of the training set and the corresponding performance parameters may be used to determine the engine model parameter value(s).

Having determined the engine model parameter values, the mechanistic engine model may be used to determine a functional relation between the molecular descriptors and the engine model parameters, thereby obtaining a functional relation between the molecular descriptors and the engine operating parameter represented by the engine model parameter.

**EP 1 785 898 A1**

Using the determined functional relation, a performance parameter value for a number of possibly suitable additives may be determined. Thereto, the molecular descriptor values of the possibly suitable additives are determined. The molecular descriptor values are used to determine the corresponding engine model parameter values using the determined functional relation. Then, using the molecular descriptor values and the engine model parameter values in the mechanistic engine model, the performance parameter value may be determined. Based on their thus determined performance parameter value, a relatively small number of the possibly suitable additives may be selected. Only the relatively small number of selected additives may be used in further experiments, such as in a bench test and/or in an engine test.

Whereas, in a known prior art method, a modeling method such as QSPR modeling is employed to determine a direct functional relation between at least one molecular descriptor and a performance parameter, in the present invention a modeling method such as QSPR modeling is employed to determine a functional relation between the molecular descriptors and at least one engine model parameter representing a property of a process occurring in the engine in order to determine the performance parameter value using the determined engine model parameter. Since the parameter(s) relate to actual processes, insight in the operation of the engine and the influence of the additives is obtained, which insight may later be used for selecting possibly suitable additives and/or for combining two or more additives.

The insight in the operation of the engine, in particular in relation to the additives, further enables to predict whether other chemical structures and/or engine parameters may be changed in order to improve the engine performance. Which parameters may be studied and changed depends on the parameters used in the mechanistic engine model part. Based on any technical consideration, the complexity of the mechanistic engine model part may be increased or decreased, or parameters may be replaced by other parameters. For example, a relatively simple mechanistic engine model part may be increased in complexity by adding models of additional physiochemical processes. Thus, the use of the mechanistic engine model allows increasing the insight into the operation of the engine.

## DETAILED DESCRIPTION OF THE INVENTION

Thus, the invention, in one aspect, relates to a method for predicting and/or correlating additive structure to engine performance. More in particular, the present invention relates to a method of determining an engine operating parameter as a function of a molecular parameter of a chemical structure, the chemical structure being for use as an additive in an engine fluid, the method comprising:

(a) providing a mechanistic engine model for estimating a value of a performance parameter of an engine, the engine model comprising an engine model parameter representing the engine operating parameter;
(b) selecting a training set of chemical structures;
(c) selecting a molecular parameter characterizing at least one property of the chemical structure;
(d) providing a molecular parameter value of the molecular parameter selected in step (c) for each chemical structure of the training set selected in step (b);
(e) providing a model parameter value of the engine model parameter for each chemical structure of the training set selected in step (b); and
(f) determining a functional relation between the molecular parameter and the engine model parameter.

In a further aspect, the present invention relates to a method of predicting a performance parameter value of a performance parameter of an engine as a function of a chemical structure used as an additive in an engine fluid, the method comprising:

(a) providing a mechanistic engine model for estimating the performance parameter value of the performance parameter of an engine, the engine model comprising at least one engine model parameter representing an engine operating parameter;
(b) determining an engine model parameter value for each engine model parameter for the chemical structure using a functional relation between at least one molecular parameter of the chemical structure and the at least one engine model parameter, the functional relation being obtained in accordance with the above-mentioned method according to the present invention;
(c) predicting the performance parameter value using the mechanistic engine model comprising the at least one engine model parameter value as obtained in step (b); and
(d) determining whether the chemical structure is a suitable additive based on the predicted performance parameter value.

4

# EP 1 785 898 A1

## SHORT DESCRIPTION OF THE DRAWINGS

Hereinafter the present invention is described in more detail with reference to the appended drawings showing non-limiting embodiments and in which:

Fig. 1A          schematically illustrates a prior art method of determining a performance parameter value as a function of at least one molecular descriptor of an engine fluid;

Fig. 1b          schematically illustrates a method according to the present invention for determining a performance parameter value as a function of at least one molecular descriptor of an engine fluid;

Fig. 2A          schematically illustrates an operation of an engine;

Fig. 2B          schematically illustrates a part of an engine;

Fig. 2C          schematically illustrates a mechanistic engine model based on the engine part of Fig. 2B;

Fig. 2D          schematically illustrates a reaction kinetics model; and

Fig. 3A and 3B   schematically illustrate an embodiment of the method according to the present invention.

## DETAILED DESCRIPTION OF THE DRAWINGS

In the drawings, like reference numerals refer to like elements.

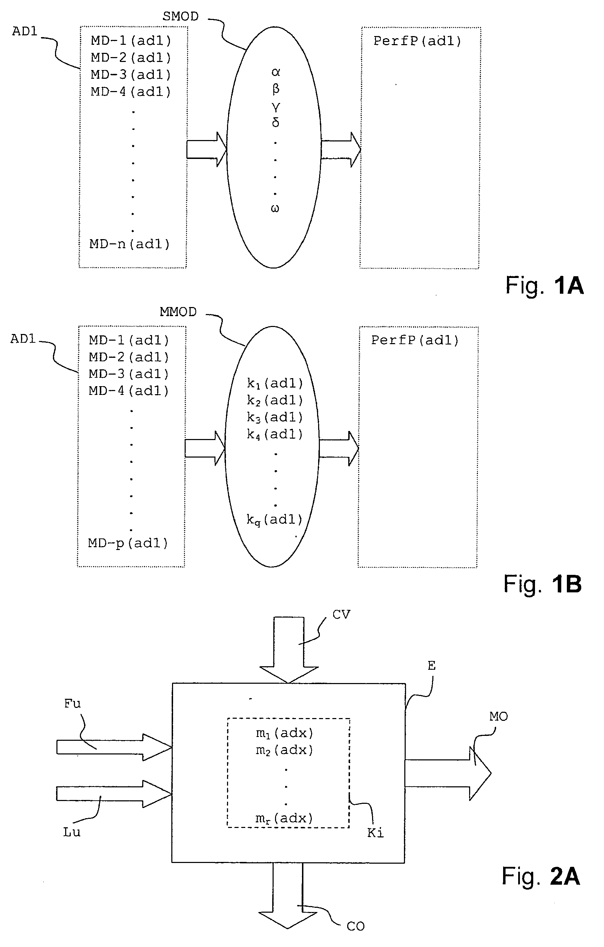

Fig. 1A illustrates a prior art method for determining a functional relation between a first additive AD1 and a corresponding performance parameter value PerfP(ad1). The first additive AD1, i.e. a molecule having a molecular structure to be added to another composition, is described by a number of molecular parameters known in the art as molecular descriptors MD-1(adl) - MD-n(adl). The number (n) of the molecular descriptors MD used in the method may be selected by a user, possibly depending on statistical or technical considerations.

A statistical model SMOD comprises a number of statistical model parameters $\alpha$ - $\omega$. Based primarily on mathematical considerations, the statistical model SMOD comprises a number of equations comprising the statistical model parameters $\alpha$ - $\omega$. In order to determine a value for each of the statistical model parameters $\alpha$ - $\omega$, a training set of additives, e.g. comprising the first additive AD1, is selected. A corresponding value of the performance parameter PerfP, e.g. the performance parameter value PerfP(ad1) relating to the first additive AD1, is determined. The performance parameter value may be determined from a database or literature, or the performance parameter value may be determined using suitable experiments, such as engine tests. Using the molecular descriptor values of the additives comprised in the training set, the corresponding performance parameter values, the statistical model SMOD and a suitable estimation method such as Partial Least Squares regression modelling, a value for each statistical model parameter $\alpha$ - $\omega$ may be obtained.

Having obtained a value for each statistical model parameter $\alpha$ - $\omega$, a performance parameter value PerfP for any other additive, i.e. any additive not comprised in the training set, may be estimated or predicted using the value of the molecular descriptors MD-1 - MD-n of the additive, the values of the statistical model parameters $\alpha$ - $\omega$ and the statistical model SMOD.

In the above-described prior art method the statistical model parameters are merely mathematical parameters. The statistical model parameters do not provide any information about or insight into the operation of the device that is modeled, e.g. an engine.

Fig. 1B illustrates an embodiment of a method according to the present invention for determining a functional relation between a first additive AD1 and a corresponding performance parameter value PerfP(ad1). In accordance with the above-described prior art method, the first additive AD1 is described by a number of molecular parameters known in the art as molecular descriptors MD-1(ad1) - MD-n(ad1). The number n of the molecular descriptors MD used in the method may be selected by a user, possibly depending on statistical or technical considerations.

A mechanistic model MMOD comprises a number of mechanistic model parameters $k_1$ - $k_q$. Based primarily on technical considerations, the mechanistic model MMOD comprises a number of equations comprising the mechanistic model parameters $k_1$ - $k_q$. The mechanistic model parameters $k_1$ - $k_q$ relate to physical, chemical or physiochemical processes occurring in an engine. As these processes are influenced by any compound supplied, the first additive AD1 influences a value of the mechanistic model parameters $k_1$ - $k_q$. Thus, the mechanistic model parameters $k_1$ - $k_q$ are a function of the first additive AD1, as indicated in Fig. 1B ($k_1$(ad1) - $k_q$(ad1)).

In order to determine a value for each of the mechanistic model parameters $k_1$ - $k_q$, a training set of additives, e.g. comprising the first additive AD1, is selected. A corresponding value of the mechanistic model parameters $k_1$ - $k_q$, e.g. the mechanistic model parameter value $k_1$ (ad1) relating to the first additive AD1, is determined. Each mechanistic model parameter value may be determined from a database or literature, or each mechanistic model parameter value may be determined using suitable experiments, such as a bench test. Using the molecular descriptor values of the additives comprised in the training set, the corresponding mechanistic model parameter values, and a suitable statistical model, e.g. a linear model, and a suitable model parameter estimation method, such as Partial Least Squares regression, a value for each mechanistic model parameter $k_1$ - $k_q$ as a function of the molecular descriptors of an additive may be obtained. The development of these functions is equivalent to the development of a Quantitative-Structure Performance Relationship (QSPR) for each mechanistic model parameters.

Having obtained a functional relationship between the molecular descriptors and the mechanistic model parameters, a performance parameter value may be determined. Further, insight into the operation of the engine is obtained. If an additive gives a good or a poor performance parameter value, the corresponding mechanistic model parameters for the additive may be studied. Based on such studies, a change in a performance parameter value may be predicted for a combination of additives. For example, one additive may improve a first mechanistic model parameter; another may improve a second mechanistic model parameter.

Further, for a chemical structure that is not part of the training set, a suitable set of molecular descriptor values may be selected. For example, using Principal Component Analysis (PCA), a set of significant molecular descriptors may be selected and a desired value thereof may be determined. Then, a chemical structure having the desired value of the set of significant molecular descriptors may be selected from a library or may be synthesized.

For illustrating a mechanistic engine model, in Fig. 2A, an engine E is schematically illustrated. As an input, the engine E receives a fuel Fu and is lubricated using a lubricant Lu. Other fluids, or the like, may be input in the engine E for the engine to operate. As an output, the engine E provides a mechanical output MO e.g. for moving an object such as a car. However, the engine E may be employed in other applications as well.

In modern engines E, the engine E may be controlled by an electronic control system which supplies control variables CV to the engine E. The control variables CV may control any operating parameter of the engine E, possibly as a function of a determined value of another operating parameter. In general, the control system determines and supplies the control variables CV in order to increase a performance of the engine E.

Apart from a mechanical output MO, the engine E as well has a chemical output CO. Possibly, further outputs may be defined as well.

Inside the engine E, the fuel Fu is used in an exothermal chemical reaction. Some of the energy that is released is supplied as a mechanical output. The chemical reaction in the engine E may be defined or modeled by a number of equations using a number of operating parameters. For example, the kinetics Ki of the chemical processes may be defined by a number of kinetic operating parameters $m_1$ - $m_r$.

In order to increase the performance of the engine E, an additive adx may be used. The additive adx may be added to the fuel Fu or the lubricant Lu, or any other engine fluid. The additive adx thus enters in the chemical processes occurring in the engine E. Hence, the additive adx influences the kinetics Ki. Consequently, the kinetic operating parameters $m_1$ - $m_r$ are a function of the additive adx. However, the engine model as illustrated in Fig. 2A does not provide any information as to how the properties of the molecular structure of the additive adx influence the engine performance.

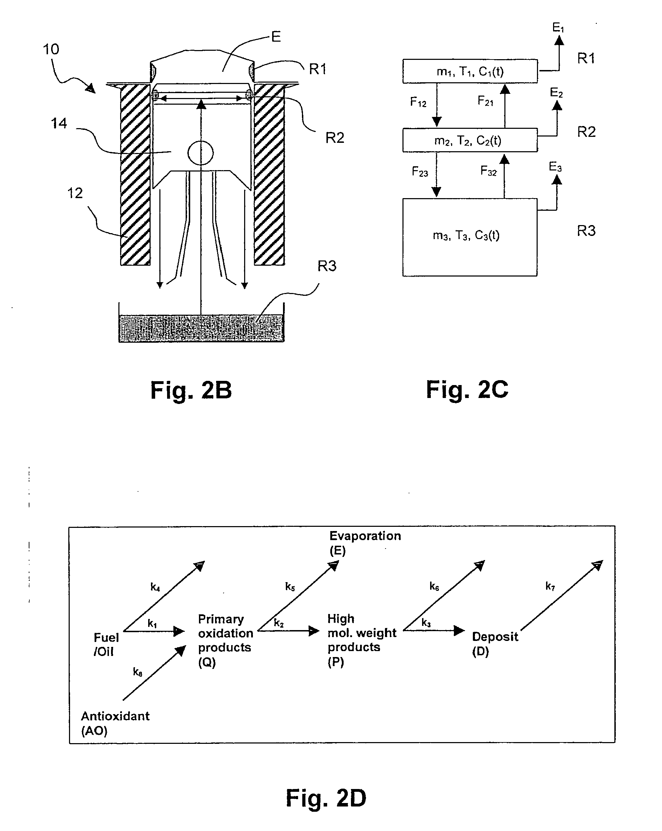

In Fig. 2B an engine piston-cylinder assembly 10 is illustrated. The piston-cylinder assembly 10 comprises a cylinder 12 and a piston 14. The operation of the piston 14 in the cylinder 12 is well known in the art.

In the cylinder 12 three reactors R1 - R3 may be identified. A first chemical reactor R1 may be identified at the cylinder liner - piston ring interface. A second chemical reactor R2 may be identified at a top ring zone. A third chemical reactor R3 may be identified in the oil sump.

In Fig. 2C a mechanistic model of the three chemical reactors R1 - R3 is illustrated. The three reactors R1 - R3 are coupled as the compounds to be used as an input in a first reactor R1 - R3 depend on an output of a second reactor R1 - R3, as indicated by the mass flow rate arrows $F_{12}$, $F_{23}$, $F_{32}$, $F_{21}$. Further, from each reactor R1 - R3, some compounds may evaporate as indicated by arrows E1 - E3. In each reactor R1 - R3, separate conditions may be present, such as a reactor capacity $m_1$ - $m_3$, an average reaction temperature T1 - T3, and a concentration of a species $C_1(t)$ - $C_3(t)$ as a function of time t.

Fig. 2D illustrates an example of a kinetics model describing the reaction kinetics Ki (as indicated in Fig. 2A) of a reaction occurring in at least one of the reactors R1 - R3 (as indicated in Fig. 2B and 2C).

Referring to Fig. 2D, the mechanistic kinetics model encompasses a reaction model comprising reaction rate equations and kinetic parameters. For each reactor R1 - R3 in the engine model such a set of equations can be developed. The model depicted in Fig 2D is a model for the oxidation and degradation of a lubricant comprising a lubricating component and an additive in an engine. For this model, the kinetics can be described with a set of rate equations:

Original Oil (RH):

$$\frac{dM_{RH}}{dt} = -k_1 M_{RH} - k_4 M_{RH} \qquad \text{(Eq. 1)}$$

Primary Oxidation product (Q):

$$\frac{dM_Q}{dt} = k_1 M_{RH} + k_8 M_{AO} - k_5 M_Q - k_2 M_Q \qquad (\text{Eq. 2})$$

High Molecular weight product (P):

$$\frac{dM_P}{dt} = k_2 M_Q - k_3 M_P - k_6 M_P \qquad (\text{Eq. 3})$$

Deposit (D):

$$\frac{dM_D}{dt} = k_3 M_P - k_7 M_D \qquad (\text{Eq. 4})$$

Evaporation (E):

$$\frac{dM_E}{dt} = k_4 M_{RH} + k_5 M_Q + k_6 M_P + k_7 M_D \qquad (\text{Eq. 5})$$

Additive (AO):

$$\frac{dM_{AO}}{dt} = k_8 M_{AO} \qquad (\text{Eq. 6})$$

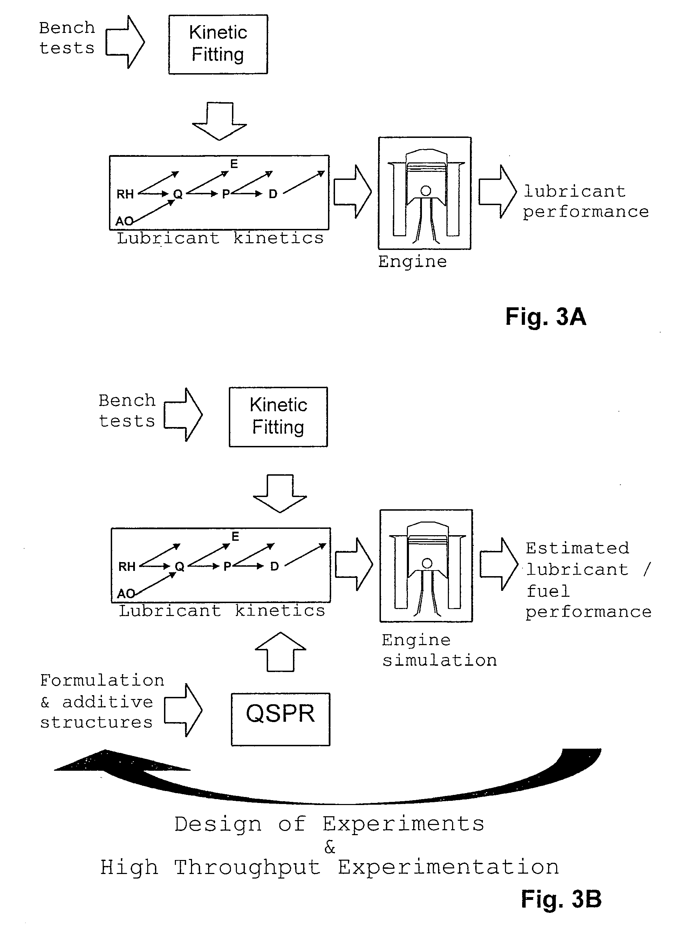

Hereinafter, a more detailed description of the method according to the present invention, and embodiments thereof, is given with reference to Figs. 2A - 2D and Figs. 3A - 3B. Fig. 3A shows a schematic representation of an embodiment comprising the prediction of lubricant performance based on an engine simulation where the engine model parameters are determined using data from bench tests and kinetic fitting based on engine performance data. Fig 3B shows a schematic representation of the embodiment of Fig. 3A, comprising the prediction of lubricant performance based on an engine simulation where the engine operating parameters are estimated for novel additives and formulations using a QSPR model to estimate changes in the engine model parameters as a function of changing the molecular structure of an additive, specifically the molecular parameters characterising the novel additives. An iterative cycle of high-throughput experimentation and modelling can then be used to optimize additive performance.

In the method according to the present invention, an engine model is provided. Preferably the engine model comprises a set of interconnected reactor models that model the whole engine or selected parts of the engine, such as the part where the most relevant chemical reactions are taking place. Examples thereof are the above-described cylinder liner - piston ring interface, the oil sump, the top ring zone etc. Each part of the engine can be described as a separate chemical reactor, characterised by its own set of parameters as capacity, temperature, concentrations (reactant compositions), mass flow rates etc. The separate chemical reactors can be assembled to an interconnected set of reactors that represent the engine or a part thereof.

In accordance with an embodiment of the present invention a kinetic model comprising kinetic model parameters of reaction kinetics in an engine is provided. The kinetic model may encompass a reaction model comprising reaction rate equations and kinetic model parameters. For each reactor model in the engine model such a set of equations can be developed. An example of such a model is depicted in Fig. 2D for the oxidation and degradation of a lubricant comprising a lubricating component and an additive in an engine. For this model, the kinetics can be described with the above-described set of rate equations.

In an embodiment, the rate equations for various individual components in the lubricant or fuel and the various reaction products produced can be selected from the group consisting of the fuel/lubricant rate equation, the additive rate equation, the evaporation rate equation, the deposit rate equation, the high Mw product formation rate equation, etc.

The reaction equations can be embedded in a multiple reactor system model described by a set of ordinary and differential equations. Such a reactor system can be configured to model an engine by choosing appropriate reactor temperatures, volumes, residence times and flow rates between the reactors. Such an approach to modelling a system of chemical reactors is in itself well known in the reaction engineering literature. One example is for instance disclosed in the article of Chen et al. Tribology letters 1990, 14(2), 83 ff. albeit without any reference to apply this in an environment to predict performance based on QSPR modelling.

The additives used in the method according to the present invention are characterised using a set of molecular descriptors. Typically each additive is characterised using a set of molecular descriptors. Molecular descriptors in themselves are known, and are used to describe single molecular species properties. Typical molecular descriptors can be based on bulk physical properties such as boiling points, critical temperature, vapour pressure, flash point, auto-ignition temperature, density, refractive index, melting point, octanol-water partition coefficient, aqueous solubility of liquids and solids, molecular mass, water-air partition coefficients, GC retention times and response factor, critical micelle concentration, polymer glass transition temperature, polymer refractive index, and molecular descriptors such as partial charge and charge densities, dipole moment, molecular surface area, molecular volume, electrostatic potential, bond lengths, bond angles, heat of formation, hydrogen bonding ability, etc. See also Katritzky et al-. Chem. Inf. Comput. Sci, 2000, 40, 1-18 and references cited therein on descriptor generation. Molecular descriptors can also include hash-key or structure fingerprints, constitutional descriptors such as functional group counts, fragment contribution, atomic contributions, topological descriptors such as connectivity indices, Wiener numbers and Balaban indices or geometric descriptors such as, solvent excluded volume and WHIM descriptors. In certain embodiments, typically in cases where novel compounds are to be designed and no bulk physical parameters are available, the generation of some molecular descriptors may require molecular modelling using, for example, molecular mechanics, semi-empirical or quantum mechanical methods.

In general, molecular descriptors provide a set of parameters that allow adequate description of changes in the molecular structure.

With a number of additives a plurality of compositions are formulated. Preferably, the design of the compositions is such that the desired experimental space is covered, meaning that, based on for instance statistical analysis and cheminformatics, the compositions to be tested, are selected such that the set to be tested contains a representative number and variation of the different additives. The characteristics typically match the range of additives that are contemplated, not only for testing, but mainly for predicting in a later stage of the method. The plurality of compositions can also be determined using an automated or computerised design of experiments.

It is also possible to develop a combinatorial library of compositions to be tested, each member of the combinatorial library comprising one or more additives in varying amounts. The plurality of compositions can be formulated in high throughput fashion using highly automated equipment such pippetting robots etc.

The different compositions may be tested under controlled circumstances, either in laboratory bench tests or in engine tests, depending on the circumstances, and the relevant performance parameters are determined. Typical performance parameters are wear, deposit formation, degradation, soot formation etc. A wide variety of technologies are known in the field and novel ones are developed everyday. Given the engine model, suitable test procedures can be selected that provide for the rate constant in a reliable manner. As engines typically function over a range of temperatures, the Arrhenius equation can be used to accommodate for the change of the reaction constant as a function of the temperature. Possible technologies for evaporation under engine operating conditions can be thermal gravimetric analysis. Oxidation stability can be measured using DSC. For formation of high molecular weight products and deposit rate constants, a modified micro-oxidation test can be used according to Naidu et al. Ind. Eng. Chem. Prod. Res. Dev., 1986, 25, 596 etc.

In certain embodiments, the testing is, preferably simultaneously, performed in a plurality of laboratory bench tests and/or engine tests, preferably laboratory bench tests.

The performance parameters may be used to determine and/or to estimate the kinetic model parameters in the model such that the variation in the compositions and the effect on the performance is reflected in the determination of the kinetic model parameters. In certain embodiments, the performance parameters are obtained by directly measuring the kinetic model parameters in bench tests and/or engine tests. The kinetic model parameters can also be obtained by kinetic fitting to the results of bench tests.

Preferably, the kinetic model parameters are determined simultaneously, using high throughput methodologies such as kinetic fitting. This may involve numerically integrating a set of ordinary and differential equations describing the experiments and using an optimisation algorithm to select kinetic model parameters that provide the best fit to the measured composition and performance parameters. An appropriate numerical integration algorithm may include a Runge-Kutta method, though preferably a method that is robust to stiff systems of equations will be used preferably an algorithm based on Gear's method. Appropriate optimisation algorithms for the determination of the kinetic model parameters include Gauss-Newton and Levenburg-Marquardt methods and includes solving the various rate equations discloses herein and determination of the kinetic model parameters for a variety of additives and formulations. Together with a statistical analysis of the various rate constants for the various compositions, this provides a relation between the structure of the additive and the associated rate constants. The advantage of the fitting of the kinetic model parameters resides in the ability to absorb poorly understood effects. It is clear from the above that the present method in itself is not limited to the set of rate equations exemplified herein, but that for a different set of equations, a similar approach can be applied.

Kinetic fitting of bench test data for a range of additive/lubricant compositions may be used to determine the kinetic model parameters for each of these compositions. Statistical analysis can then be used to develop a QSPR between the structures of the additive/lubricant components, in particular, one or more molecular descriptors of these additives/components, and the identified model kinetic parameters, resulting in a Quantitative Structure Performance Relationship (QSPR) model. It will be clear that a Quantitative Structure Performance Relationship can also be a Quantitative Structure Kinetics Relationship (QSKR), depending on whether the performance parameters or the kinetic parameters are used in the development of the quantitative relation. It is noted that a QSPR or a QSKR is not conceptually different from a QSAR (Quantitative Structure Activity Relationship) such as is known from pharmaceutical fields. Such QSARs are described in the literature, and also for lubricant or fuel development, but not with respect to performance parameters or kinetic model parameters.

Performance in an engine (a laboratory test engine or actual engine) can then be determined by simulation in an engine model, for example by numerically integrating the ordinary and differential equations comprising the engine/kinetic model.

The QSPR in combination with the engine model provides a quantitative relationship between the structure of an additive/lubricant, in particular one or more of the molecular descriptors, and the performance (or the kinetic model parameters) of the engine (laboratory test engine or actual engine).

Once a QSPR model is developed, typically based on a limited number of experiments or a limited set of available data, predictions can be made between potential additives and their corresponding kinetic model parameters and performance in lubricant compositions. Also vice versa, when a certain performance of a lubricant or additive is desired, the corresponding performance parameter can be determined and, based on the associated kinetic model parameters, the desired variation in molecular descriptors can be determined, leading to a number of suggestions on additive variations that based on the model may lead to the desired improvement of the performance parameter. These methods allow for the prediction of the effect of certain additives when these additives have not yet been actually tested, but have only been simulated in silico. Such an algorithm is described for instance in applicant's co-pending application EP1589463.

Thus, in certain embodiments, the performance of one or more other additives in a laboratory bench test and/or an engine test is predicted by predicting the kinetic parameters associated with the other additives using the QSPR and the engine/kinetic model. This can be achieved using an optimiser to find a set of kinetic parameters that meet a performance target. This means solving the inverse QSPR problem to find an additive structure that may give the desired kinetic parameters. One possible solution for this problem is described in EP1589463.

Preferably, the tests on which the QSPR is developed are laboratory bench tests. Nevertheless, once actual engine test data are obtained, the QSPR can be supplemented with these data to develop further and more accurate relations.

It is possible, for instance in the case of the availability of data that have been obtained in the scope of other experiments to create a so-called virtual library. These data from earlier tested additives can be coupled to molecular descriptors and the earlier test data can function as the performance parameters, thereby allowing the determination of the underlying kinetic parameters. These data can be used to help build the model and to further aid in developing a QSPR. Also, once predictions have been made for the performance or associated kinetic parameters for certain novel additives, the actual data once the novel additives have been tested can be supplemented to the QSPR, thereby further helping in developing the model and to improve the accuracy of the predictions. This supplementation of earlier or later obtained performance data, whether in the laboratory bench testing state or in the actual engine testing state, also aids in the improvement of the quality of the subsequent predictions, the same way as the development and incorporation of later developed molecular descriptors does.

The prediction of suitable additives can be further improved by providing a model of the reactor (engine) and to provide a kinetic model that is capable of accommodating the various stages in the engine where the additive is taking its effect and the associated kinetics. The engine itself can be modelled in the form of a series of reactors as depicted in Fig 2C.

A detailed description of the reaction kinetics of a complex mixture of lubricant with additives reacting under a wide range of reacting conditions is difficult. Simplification is preferred. Instead of treating individual species formation as a basis of reaction kinetic equation description, a broad class of reaction products can be used. Such a reaction model is shown in Fig. 2D. The lubricant (RH) evaporates ($k_4$) and reacts with oxygen ($k_1$) to form the primary oxidation products (Q). The oxidised products also evaporate and further oxidise to form high-molecular weight products (P), which eventually form deposits (D). The extent of reactions is determined by the relative magnitudes of the rate constants (k's). Therefore, rate equations that describe the reaction system can be derived and are shown in the figure. When the rate constants can be independently determined, the resulting partial differential equations (with respect to time) can be solved numerically to provide the amount of each component at a given engine simulation point in time.

The engine is simulated by a number of reactors. The number of reactors in the system is determined by the engine design and usually matches the number of critical regions in the engine. Each region will define a set of environmental conditions for that reactor. See Figs. 2B and 2C. The reactor is defined by the capacity of the reactor, the temperature, and the residence time. The characteristics of reactors are determined by the dimension of the engine and the actual operating conditions. The physical properties of the oils, such as viscosity, may also affect the capacity of the cylinder liner/piston ring interface (reactor) through variation in oil-film thickness. When the characteristics of a reactor and the lubricant are defined, simulation technology can be used to calculate the reaction products for a reactor as a function of time based on the kinetic equations outlined hereinbefore. Thus, for each reactor, a similar set of rate equations can be formulated. Other aspects of the engine operation such as oil flow rates among the reactors and the degree of mixing between the reactants and products in each reactor can also be taken into account or suitable assumptions, such as steady state flow rates and/or ideal mixing behaviour, can be made.

In certain embodiments, three reactors are used to represent the engine operation as illustrated in Fig. 2C. The cylinder line/piston ring interface (the first reactor R1), the top ring groove and the volume above the ring (the second reactor R2) and the oil sump (the third reactor R3), as shown in the figure. In first reactor R1, the average temperature per stroke cycle is very high (250-300°C) but the oxygen supply is usually limited due to combustion in the cylinder and the need for oxygen to diffuse into the oil film. Evaporation here is significant. In the second reactor R2, the residence time of the oil is much longer and the temperature is high (lower than the first reactor R1). With abundant oxygen, oxidative degradation reactions are rapid and deposits will accumulate. In the sump, the temperature is relatively low. The initiation step of oxidation is not likely to take place here. However, with the free radicals generated in the ring zone, a significant amount of sludge can form in the sump. The capacity (m), the average reaction temperature (T), and the residence time ($t_{resd}$) of each reactor are determined by specific engine designs. The concentration of a species C(t) at any time (t) is determined by the kinetics of the reactions.

The oil-flow path is divided into two separate loops. The first reactor R1 and the second reactor R2 constitute one loop. The second reactor R2 and the third reactor R3 constitute another loop. This is to simulate the engine situation. The oil is first pumped from the oil sump (the third reactor R3) to the top piston ring zone (second reactor R2), where the oil resides for several minutes. During this time, the oil circulates between the ring zone (second reactor R2) and the cylinder liner/piston ring interface (first reactor R1) continuously as the piston goes up and down. When the residence time of the oil in the ring zone (second reactor R2) is reached, the oil leaves the ring zone and flows back to the sump (third reactor R3). Each time the lubricant leaves a reactor, mass balance among the three reactors is performed to account for the evaporation and deposit accumulation, if any. The reacted lubricant then becomes the feed to another reactor and undergoes further reactions. The oil transport between reactors is governed by the residence times in each reactor. The amount of oil exchanged will be determined by the oil pumping rate and mass balance under steady-state operation. Perfect mixing in each reactor is preferably assumed after oil exchange. The change in physical properties of the lubricant can be accommodated each time the mass balance is performed.

The necessary engine parameters needed such as the volume of the reactor, the temperature and residence time can be obtained either directly from the design of the engine (such as sump dimensions, oil pump rate, ring zone gap volume, or may need estimation or actual measurement.

Based on the above description, it is clear that, given the availability of a model of the kinetics in the engine, this development of a QSPR model based on kinetic data is applicable not only to lubricant additives, but is likewise applicable to fuel additives and fuel components.

In certain embodiments, the fuel is selected from the group consisting of motor fuels, preferably diesel fuel and/or gasoline, kerosene, jet fuels, marine bunker fuel, natural gas, home heating fuel and mixtures thereof.

In certain embodiments relating to fuel additives, the fuel additive can be selected from the group consisting of detergents, cetane improvers, octane improvers, emission reducers, antioxidants, carrier fluids, metal deactivators, lead scavengers, rust inhibitors, bacteriostatic agents, corrosion inhibitors, antistatic additives, drag reducing agents, demulsifiers, dehazers, anti-icing additives, dispersants, combustion improvers and the like and mixtures thereof.

In certain embodiments relating to lubricant additives, the additives can be selected from the group consisting of antioxidants, anti-wear agents, detergents, rust inhibitors, dehazing agents, demulsifying agents, metal deactivating agents, friction modifiers, pour point depressants, antifoaming agents, co-solvents, package compatibilisers, corrosion-inhibitors, ashless dispersants, dyes, extreme pressure agents and mixtures thereof.

In certain embodiments, the method can be used for the screening of lubricant compositions comprising base oil and additives for compatibility with elastomers in terms of tensile strength measurement, selected from the group consisting of olefinic elastomers, styrenic elastomers, poly (ether/ester) elastomers, polyacrylate elastomers, natural rubbers, synthetic rubbers, elastomer seals and mixtures thereof.

In certain embodiments, the method can be used for determining deposit formation tendencies of additives and/ or for determining dispersancy performance in the additional presence of a lubricant insoluble material.

In certain embodiments, the method is performed under program control, i.e. software is used to carry out all or parts of the method, in particular relating to the flow of data, generation of molecular descriptors using calculational means such as modelling software, describing the kinetic model in terms of the rate equations and/or the engine model in terms of the interconnected model reactors, determination of the relation between the molecular descriptors and the kinetic parameters, the development of the QSPR model, the prediction of performance of novel additives based on the QSPR model and so on. Accordingly, the invention encompasses a computer program that, when it is run on a computer, is capable of exercising parts or all steps of the method. From the detailed description of the method herein, the particular steps that can be performed by a computer program can be easily determined and accomplished. It is also understood that a library of additives as well as a set of molecular descriptors of each additive also means a set of data that can be used as input data in a computer program.

Typical examples of lubricants and additives are disclosed in WO2005/079277, WO2005/079278, WO2005/079279, WO2005/079280.

In accordance with the present invention the mechanistic engine model can be used for prediction of novel additive and/or lubricant/fuel formulation performance. The additive structures are characterised by molecular descriptors and/or other properties. A correlation is achieved by determining the lubricant/fuel kinetic parameters directly from bench tests or by fitting kinetic parameters to the results of engine tests, development of lubricant/fuel and/or additive structure-property relationships (QSPRs) for predicting lubricant/fuel kinetic parameters, predicting kinetic parameters for novel additives in lubricant and/or fuel formulations and based on these predictions, simulating the performance in an actual engine. This typically involves modelling the lubricant or fuel reactions in an engine or a part of the engine, determining the lubricant or fuel kinetics, simulating the lubricant or fuel performance in a laboratory bench test or in an actual engine, testing the lubricant or fuel performance in a laboratory bench test or in an actual engine, developing lubricant/fuel and/or additive structure-kinetic relationships in a QSPR, and based on the relationships developed in the QSPR model and the engine simulation, predicting the performance of novel additives in lubricant/fuel formulations and/or, given a desired performance or modification in performance, predicting and designing the structure of potential additives for lubricants and/or fuels.

Although detailed embodiments of the present invention are disclosed herein, it is to be understood that the disclosed embodiments are merely exemplary of the invention, which can be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure. Further, the mere fact that certain measures are recited in mutually different dependent claims does not indicate that a combination of these measures cannot be used to advantage.

Further, the terms and phrases used herein are not intended to be limiting; but rather, to provide an understandable description of the invention. The terms "a" or "an", as used herein, are defined as one or more than one. The term another, as used herein, is defined as at least a second or more. The terms including and/or having, as used herein, are defined as comprising (i.e., open language).

**Claims**

1. Method of determining an engine operating parameter as a function of a molecular parameter of a chemical structure, the chemical structure being for use as an additive in an engine fluid, the method comprising:

   (a) providing a mechanistic engine model for estimating a value of a performance parameter of an engine, the engine model comprising an engine model parameter representing the engine operating parameter;
   (b) selecting a training set of chemical structures;
   (c) selecting a molecular parameter characterizing at least one property of the chemical structure;
   (d) providing a molecular parameter value of the molecular parameter selected in step (c) for each chemical structure of the training set selected in step (b);
   (e) providing a model parameter value of the engine model parameter for each chemical structure of the training set selected in step (b); and
   (f) determining a functional relation between the molecular parameter and the engine model parameter based on the parameter values as provided in steps (d) and (e).

2. Method according to claim 1, wherein the chemical structure is selected from a library, the library being an actual library, a combinatorial library, a virtual library or a combination thereof.

3. Method according to claims 1 or 2, wherein the chemical structure is selected from the group consisting of lubricant additives, lubricant components, fuel additives, fuel components.

4. Method according to claim 3, wherein the fuel is selected from the group consisting of motor fuels, kerosene, jet fuels, marine bunker fuel, natural gas, home heating fuel and mixtures thereof.

5. Method according to claim 4, wherein the motor fuels are selected from the group consisting of diesel fuel and gasoline.

6. Method according to claims 3 to 5, wherein the at least one fuel additive is selected from the group consisting of detergents, cetane improvers, octane improvers, emission reducers, antioxidants, carrier fluids, metal deactivators, lead scavengers, rust inhibitors, bacteriostatic agents, corrosion inhibitors, antistatic additives, drag reducing agents, demulsifiers, dehazers, anti-icing additives, dispersants, combustion improvers and the like and mixtures thereof.

7. Method according to claims 3 to 6, wherein the lubricant additives are selected from the group consisting of antioxidants, anti-wear agents, detergents, rust inhibitors, dehazing agents, demulsifying agents, metal deactivating agents, friction modifiers, pour point depressants, antifoaming agents, co-solvents, package compatibilisers, corrosion-inhibitors, ashless dispersants, dyes, extreme pressure agents and mixtures thereof.

8. Method according to any of the previous claims, wherein the molecular parameters are developed by a molecular modelling approach.

9. Method according to any of the previous claims, wherein the molecular parameters are selected from the group consisting of boiling point, critical temperature, vapour pressure, flash point, auto-ignition temperature, density, refractive index, melting point, octanol-water coefficient, fragment contribution, atomic contributions, partial charge and charge densities, dipole moment, molecular surface area, molecular volume, electrostatic potential, bond length, bond angle, heat of formation, hydrogen bonding ability, aqueous solubility of liquids and solids, molecular mass, water-air partition coefficient, GC retention time and response factor, critical micelle concentration, polymer glass transition temperature, polymer refractive index, hash-key or structure fingerprints, constitutional descriptors such as functional group counts, topological descriptors such as connectivity indices, Wiener numbers and Balaban indices or geometric descriptors such as molecular surface area, solvent excluded volume and WHIM descriptors

10. Method according to any of the previous claims, wherein the mechanistic engine model comprises a reaction kinetics model.

11. Method according to any of the previous claims, wherein the reaction kinetics model is a model describing the lubricant degradation kinetics of an engine and/or the fuel combustion kinetics of an engine.

12. Method according to any of the previous claims, wherein step (e) further comprises:

   (e1) providing a value of a performance parameter of the engine for each chemical structure of the training set selected in step (b); and
   (e2) determining a value of an engine model parameter based on performing a bench test.

13. Method according to any of the previous claims, wherein step (e) further comprises:

   (e1) providing a value of a performance parameter of the engine for each chemical structure of the training set selected in step (b); and
   (e3) determining a value of an engine model parameter using an optimisation algorithm, based on a performance parameter value as provided in step (e1) and an engine model provided in step (a).

14. Method according to claim 12, wherein the engine performance is determined by an engine test in a laboratory test engine, an actual engine or a combination thereof.

15. Method according to any of the previous claims wherein testing is, preferably simultaneously, performed in a plurality of laboratory bench tests and/or engine tests, preferably laboratory bench tests.

16. Method according to any of the previous claims, wherein the performance parameter is obtained by directly measuring the engine operating parameters in bench tests or engine tests, a measured operating parameter value being used as the model parameter value.

17. Method according to any of the previous claims, wherein the performance of other chemical structures in a laboratory bench test and/or an engine test is predicted by predicting the engine model parameters using the functional relation, in particular a QSPR, as obtained in step (f).

18. Method according to any of the previous claims, wherein the engine model parameters are rate constants.

19. Method according to any of the previous claims, wherein the mechanistic engine model comprises rate equations.

20. Method according to any of the previous claims, wherein the engine is modelled by one or more interconnected chemical reactors.

21. Method according to claim 20, wherein the chemistry in each reactor is simulated by a kinetic model comprising rate equations and rate constants.

22. Method according to claim 19 or 21, wherein the rate equations are the fuel/lubricant rate equation, the additive rate equation, the evaporation rate equation, the deposit rate equation, the high molecular weight product formation rate equation.

23. Method according to any of the preceding claims, the method further comprising:

- selecting a chemical structure to be used as an additive in an engine fluid, comprising:
- determining an engine model parameter value for each engine model parameter for the chemical structure using the functional relation between the at least one molecular parameter of the chemical structure and the at least one engine model parameter,
- estimating the performance parameter value using the mechanistic engine model comprising the estimated engine model parameter value;
- determining whether the chemical structure is a suitable additive based on the estimated performance parameter value.

24. Method of selecting a chemical structure to be used as an additive in an engine fluid, the method comprising:

(a) providing a mechanistic engine model for estimating the performance parameter value of the performance parameter of an engine, the engine model comprising at least one engine model parameter;
(b) determining an engine model parameter value for each engine model parameter for the chemical structure using a functional relation between at least one molecular parameter of the chemical structure and the at least one engine model parameter, the functional relation being obtained in accordance with the method of claim 1;
(c) estimating the performance parameter value using the mechanistic engine model comprising the at least one engine model parameter value as obtained in step (b); and
(d) determining whether the chemical structure is a suitable additive based on the estimated performance parameter value.

25. Use of a QSPR model for engine performance estimation of a fuel or lubricant additive.

26. Use according to claim 25, wherein the QSPR model is based on the fitting of reaction kinetics parameters to performance parameters.

27. Computer program, comprising a set of instructions that, when running on a computer, perform the method as defined in any of the claims 1 - 24.

Fig. **1A**

Fig. **1B**

Fig. **2A**

**Fig. 2B**  **Fig. 2C**

**Fig. 2D**

Bench tests ⇨ Kinetic Fitting

⇩

RH ⟷ Q → P ⟷ D → E (arrows), AO → 
Lubricant kinetics ⇨ Engine ⇨ lubricant performance

**Fig. 3A**

Bench tests ⇨ Kinetic Fitting

⇩

RH ⟷ Q → P ⟷ D → E (arrows), AO →
Lubricant kinetics ⇨ Engine simulation ⇨ Estimated lubricant / fuel performance

⇧

Formulation & additive structures ⇨ QSPR

Design of Experiments
&
High Throughput Experimentation

**Fig. 3B**

**European Patent Office**

**DECLARATION**

which under Rule 45 of the European Patent Convention shall be considered, for the purposes of subsequent proceedings, as the European search report

Application Number

EP 06 07 6977

| The Search Division considers that the present application, does not comply with the provisions of the EPC to such an extent that it is not possible to carry out a meaningful search into the state of the art on the basis of all claims<br><br>Reason:<br><br>A meaningful search is not possible on the basis of all claims because all claims are directed to - Mathematical methods and methods for performing mental acts - Article 52 (2)(a) and (c) EPC.<br><br>The applicant's attention is drawn to the fact that a search may be carried out during examination following a declaration of no search under Rule 45 EPC, should the problems which led to the declaration being issued be overcome (see EPC Guideline C-VI, 8.5).<br>----- | **CLASSIFICATION OF THE APPLICATION (IPC)**<br><br>INV.<br>G06F19/00<br><br>ADD.<br>C10M125/00<br>G01L3/00 |

| Place of search | Date | Examiner |
|---|---|---|
| The Hague | 13 December 2006 | Swarén, Peter |

EPO FORM 1504 (P04C37)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1589463 A
- WO 2005079277 A
- WO 2005079278 A
- WO 2005079279 A
- WO 2005079280 A

**Non-patent literature cited in the description**

- **KATRITZKY et al.** *Chem. Inf. Comput. Sci,* 2000, vol. 40, 1-18
- **NAIDU et al.** *Ind. Eng. Chem. Prod. Res. Dev.,* 1986, vol. 25, 596